# EUROPEAN PATENT APPLICATION

(11) **EP 4 413 989 A1**
(43) Date of publication of application: **14.08.2024**
(21) Application number: 24157151.2
(22) Date of filing: 12.02.2024
(51) Int. Cl.: A61K 36/00, A61P 15/00, A23L 2/14, A23L 29/00, A23P 10/28, A61K 36/28, A61K 36/324, A61K 36/48, A61K 36/746, A61K 31/015, A61K 31/365, A61K 31/405, A61P 15/02, A61K 31/164

(54) **FOOD SUPPLEMENT FOR THE TREATMENT OF VULVODYNIA**

(30) Priority: 13.02.2023 IT 202300002385
(71) Applicant: TL Pharma Consulting Srl, 65129 Pescara (PE) (IT)
(72) Inventor: Torello, Giulio, Pescara (PE) (IT)
(74) Representative: IP Sextant s.r.l.

(57) **Abstract**

Dietary supplement comprising or consisting of:
- Palmitoylethanolamide, in percentual weight comprised between 20% and 30%;
- Noni juice freeze-dried powder, in percentual weight comprised between 10% and 15%;
- Boswellia resinous extract comprising about 65% of boswellic acids, in percentual weight comprised between 10% and 15%;
- β- caryophyllene , in percentual weight comprised between 8.32% and 12.5%;
- Myrrh extract, in percentual weight comprised between 6.64% and 9.96%;
- griffonia seed dry extract comprising about 25% of 5-hydroxy-tryptophan, in percentage weight between 3.36% and 5.04%;
- parthenium dry extract comprising about 0.5% of Parthenolides, in percentual weight comprised between 3.36% and 5.04%;
- microcrystalline cellulose, in percentual weight comprised between 15.7% and 23.16%;
- silicon dioxide, in percentual weight comprised between 1.66% and 1.99%; And
- Mg salts of fatty acids, in percentual weight comprised between 1.66% and 1.99%,
in which the total percentage sum of all the aforementioned components is equal to 100%.

## Description

The present invention concerns a dietary supplement for the treatment of vulvodynia.

Vulvodynia is a gynecological disease that manifests itself as a chronic condition of vulvar pain with debilitating consequences for the health and quality of life of the woman affected ^{[1]}. It affects up to 16% of women, regardless of age or ethnicity ^{[2]} and manifests itself with constant burning, itching and irritation that can spread to the entire vulva or just part of it.

The pathophysiology of vulvodynia is currently uncertain and probably multifactorial. In fact, although inflammatory, genetic, hormonal and muscular factors are involved in the genesis of vulvodynia, the etiopathogenetic mechanisms also appear to be related to a dysregulation of the mechanisms responsible for pain perception, with an increase in nerve endings resulting from the increase in the mast cell population .

Women who suffer from it report more anxiety, fear of pain, hypervigilance, catastrophizing and depression and this can have notable consequences in their lives, with limitations in carrying out common activities of daily living ^{[3-4]}. Vulvodynia also has deleterious consequences on a couple's sexuality and intimacy and overall the psychosocial component is considerably influenced.

Current methods and products for the treatment of vulvodynia, for example described in US 2019/0117634 A1 and US 2022/0152047 A1, suffer from numerous drawbacks. The main drawback is the absence of an effective therapy that acts with a synergistic mechanism on the various factors that trigger the pathology. Not only that, the drugs and supplements available for treatment are not easy to find, have high costs, and are difficult to conserve, as they are often galenic products, they act on the symptoms at a skin level and not at a systemic level, they can induce side effects side effects which are not negligible and do not promote compliance, as they involve the administration of the treatment repeated throughout the day.

There is therefore a need to improve the state of the art in relation to the therapeutic approach of this pathology and the main aim of the present invention, therefore, is to provide a product which allows the effective treatment of vulvodynia, preventing its causes, reducing the symptoms and limiting the side effects caused by the treatment. Another purpose of the present invention is to make available an easy-to-find product for the treatment of vulvodynia, which allows women undergoing treatment to easily comply with the prescription.

The specific object of the present invention is a dietary supplement comprising or consisting of:
- Palmitoylethanolamide, in percentual weight comprised between 20% and 30%;
- Noni juice freeze-dried powder, in percentual weight comprised between 10% and 15%;
- Boswellia resinous extract comprising about 65% of boswellic acids, in percentual weight comprised between 10% and 15%;
- β- caryophyllene, in percentual weight comprised between 8.32% and 12.5%;
- Myrrh extract, in percentual weight comprised between 6.64% and 9.96%;
- Griffonia seed dry extract comprising about 25% of 5-hydroxy-tryptophan, in percentage weight between 3.36% and 5.04%;
- parthenium dry extract comprising about 0.5% of Parthenolides, in percentual weight comprised between 3.36% and 5.04%;
- microcrystalline cellulose, in percentual weight comprised between 15.7% and 23.16%;
- silicon dioxide, in percentual weight comprised between 1.66% and 1.99%; and
- Mg salts of fatty acids, in percentual weight comprised between 1.66% and 1.99%,
in which the total percentage sum of all the aforementioned components is equal to 100%.

According to another aspect of the invention, the weight percent concentration of Palmitoylethanolamide can be equal to 25%.

According to a further aspect of the invention, the weight percentage concentration of the freeze-dried Noni juice powder can be equal to 12.5%.

According to an additional aspect of the invention, the percentage weight concentration of the Boswellia resinous extract comprising about 65% of boswellic acids can be equal to 12.5%.

According to another aspect of the invention, the percent weight concentration of β-caryophyllene can be equal to 10.4%.

According to a further aspect of the invention, the percentage weight concentration of Myrrh extract, optionally in the form of oleo resin, can be equal to 8.3%.

According to an additional aspect of the invention, the weight percent concentration of Griffonia seed dry extract comprising about 25% of 5-hydroxy-tryptophan can be equal to 4.2%.

According to another aspect of the invention, the percentage weight concentration of parthenium dry extract comprising about 0.5% of Parthenolide can be equal to 4.2%.

According to a further aspect of the invention, the dietary supplement can have the form of a tablet weighing 1200 mg, said tablet consisting of:
- 300 mg Palmitoylethanolamide;
- 150 mg freeze-dried Noni juice powder;
- 150 mg of Boswellia resinous extract comprising about 65% boswellic acids;
- 125 mg of β- caryophyllene, in particular REPHYLL ^{®} beta-caryophyllene;
- 100 mg of Myrrh extract, in particular MYRLIQ ^{®} myrrh gum;
- 50 mg of Griffonia seed dry extract comprising about 25% 5-hydroxy-tryptophan;
- 50 mg of parthenium dry extract including about 0.5% parthenolides;
- 235 mg microcrystalline cellulose;
- 20 mg of silicon dioxide; and
- 20 mg of Mg salts of fatty acids.

A specific object of the present invention is also the dietary supplement as described above for use in the prevention and/or treatment of vulvodynia.

According to a further aspect of the invention, the dietary supplement can be configured to be administered, orally, in the form of two tablets per day.

According to an additional aspect of the invention, the two tablets can be taken simultaneously.

The advantages offered by the present invention are significant.

In fact, the components of the dietary supplement according to the invention act synergistically according to different mechanisms of action, namely:
- anti-inflammatory, analgesic, modulatory of neuropathic pain;
- modulatory of the mast cell response;
- mood modulator, antidepressant; as well as
- local anaesthetic,
therefore, with a single product, it is possible to treat the various factors that trigger the pathology at a systemic level and, at the same time, alleviate the symptoms.

The present invention will now be described, by way of illustration and without limitation, according to its preferred embodiments.

In the following description and in the claims, the words "equal to about" mean "equal to" according to the usual industrial tolerances.

The dietary supplement according to the invention comprises or consists of:
- Palmitoylethanolamide (PEA for short), in percentual weight comprised between 20% and 30%;
- Noni juice freeze-dried powder, in percentual weight comprised between 10% and 15%;
- Boswellia resinous extract comprising about 65% of boswellic acids, in percentual weight comprised between 10% and 15%;
- β- caryophyllene, in percentual weight comprised between 8.32% and 12.5%;
- Myrrh extract, in percentual weight comprised between 6.64% and 9.96%;
- Griffonia seed dry extract comprising about 25% of 5-hydroxy-tryptophan, in percentage weight between 3.36% and 5.04%;
- parthenium dry extract comprising about 0.5% of Parthenolides, in percentual weight comprised between 3.36% and 5.04%;
- microcrystalline cellulose, in percentual weight comprised between 15.7% and 23.16%;
- silicon dioxide, in percentual weight comprised between 1.66% and 1.99%; and
- Mg salts of fatty acids, in percentual weight comprised between 1.66% and 1.99%,
in which the total percentage sum of all the aforementioned components is equal to 100%.

According to a preferred embodiment of the invention, in the supplement, the percent weight concentration of Palmitoylethanolamide is equal to 25%. Palmitoylethanolamide is an endogenous lipid compound (amide of a fatty acid) with demonstrated analgesic and anti-inflammatory properties produced as a lipid mediator in the presence of inflammatory processes and frequently used in the treatment of neuropathic pain. It manifests its anti-inflammatory and pain-relieving action in a similar way to Cannabis, but without presenting the side and psychotropic effects. It works by inhibiting the release of pro-inflammatory mediators by mast cells. In the supplement of the present invention, Palmitoylethanolamide is added at a dosage that guarantees its therapeutic effect.

According to the aforementioned preferred embodiment of the invention, in the supplement, the percentage weight concentration of freeze-dried Noni juice powder is equal to 12.5%. Morinda citrifolia L. ( Rubiaceae ), commonly known as Noni, is a small tropical tree that grows throughout Polynesia, whose leaves, fruits, bark and roots have various antibacterial, antitumor and anti-inflammatory pharmacological properties ^{[5]}.

According to a preferred form of the invention, in the supplement, the percentage weight concentration of the Boswellia resinous extract comprising about 65% of boswellic acids is equal to 12.5%. Boswellia Serrata is a tree belonging to the Burseraceae family (genus Boswellia ) that grows in the mountainous regions of India, North Africa and the Middle East. The oleo gum resins, obtained through an incision on the trunk of the tree, contain 30-60% resin, 5-10% essential oils, soluble in organic solvents, and polysaccharides. The resinous part of Boswellia serrata contains monoterpenes, diterpenes, triterpenes, tetracyclic triterpenic acids and four main pentacyclic triterpenic acids: β- boswellic acid, acetyl-β-boswellic acid, 11-keto-p-boswellic acid and acetyl-11-keto acid-β-boswellic, responsible for the inhibition of pro-inflammatory enzymes. Of these four boswellic acids, the last is the most potent inhibitor of 5-lipoxygenase, the enzyme responsible for the release of inflammatory mediators ^{[6]}.

According to a preferred form of the invention, in the supplement, the percentage weight concentration of β-caryophyllene is equal to 10.4%. β-Caryophyllene (BCP) is a sesquiterpene that acts as a natural endocannabinoid. It is present in the essential oils of spices and medicinal plants such as black pepper, rosemary, cloves etc. According to a particularly preferred embodiment of the invention, β-caryophyllene is used in liposomal form to ensure high bioavailability, for example as in the REPHYLL^{®} brand product, marketed by the company Disproquima Srl. β-caryophyllene acts as a selective receptor agonist of CB2 cannabinoids, which also exert regulatory control in the mechanisms of inflammation and in the modulation of depression and chronic pain.

According to a preferred form of the invention, in the supplement, the percentage weight concentration of Myrrh extract, optionally in the form of oil resin, is equal to 8.3%. More specifically, Myrrh extract is the exudate produced by the bark of plants belonging to the genus Commiphora myrrha, a plant used for centuries as a healing and analgesic. The myrrh furanodienes curzerene, furanoeudesma-1,3-diene, and lindestrene are responsible for the analgesic activity of myrrh [7]. Myrrh can, in fact, be a therapeutic adjuvant in the presence of chronic painful neuro-inflammatory processes localized at the pelvic level and other painful syndromes. According to a particularly preferred embodiment of the invention, the Myrrh extract used in the supplement is known by the trade name MyrLiq ^{®}, marketed by the company A.C.E.F. S.p.A.. It is an extract of Commiphora myrrha with a standardized content of curzerene, furanoeudesma-1,3-diene and lindestrene (12.31 ± 0.05 g kg ⁻¹, 18.84 ± 0.02 g kg ⁻¹ and 6.23 ± 0.01 g kg ⁻¹, respectively), therefore a high content of total furanodienes (40.86 ± 0.78 g kg ⁻¹ ). Some studies conducted with this extract have demonstrated analgesic activity against some of the most common painful symptoms, in particular headaches, muscle pain, joint pain, lumbago, fever-related pain and menstrual cramps. A direct comparison with some of the most frequently used drugs (e.g. diclofenac, ketoprofen, ibuprofen, paracetamol, tramadol and ketorolac) reveals that MyrLiq^{®} has similar effects, although it requires a longer treatment course (20 days). In particular, painful states were significantly alleviated after treatment with 200 mg of MyrLiq ^{®}/day. No side effects were reported by the volunteers. ^{[8]}

According to a preferred form of the invention, in the supplement, the percentage weight concentration of Griffonia seed dry extract comprising about 25% of 5-hydroxy-tryptophan is equal to 4.2%. Griffonia Simplicifolia is a tropical shrub native to West Africa, whose seeds are rich in 5-hydroxy-l-tryptophan (5-HTP), a direct precursor in the synthesis of serotonin (5-HT). Since vulvodynia is a multifactorial pathology in which there is a psychosomatic component, this active component is used with the aim of promoting a mood-modulating, anxiolytic and antidepressant effect, without producing sedative effects.

According to a preferred form of the invention, in the supplement, the percentage weight concentration of parthenium dry extract comprising about 0.5% of Parthenolides is equal to 4.2%. The active components responsible for the anti-inflammatory effect exerted by the dry extract of feverfew are Parthenolides (sesquiterpene lactones) which, based on the results of numerous studies, appear to have an analgesic, anti-inflammatory and anti-allergic effect.

As regards the method of preparing the dietary supplement of the invention, this can be prepared according to any conventional technique, such as for example mixing the individual components.

According to a particularly preferred embodiment of the invention, the supplement is taken in the form of a tablet, optionally weighing about 1200 mg, therefore comprising:
- 300 mg Palmitoylethanolamide;
- 150 mg freeze-dried Noni juice powder;
- 150 mg of Boswellia resinous extract comprising about 65% boswellic acids;
- 125 mg of β- caryophyllene , in particular REPHYLL ^{®} beta-caryophyllene ;
- 100 mg of Myrrh extract, in particular MYRLIQ ^{®} myrrh gum;
- 50 mg of Griffonia seed dry extract comprising about 25% 5-hydroxy-tryptophan;
- 50 mg of parthenium dry extract including about 0.5% parthenolide;
- 235 mg microcrystalline cellulose;
- 20 mg of silicon dioxide; And
- 20 mg of Mg salts of fatty acids.

The dietary supplement of the invention is advantageously used for the prevention and/or treatment of vulvodynia and can be taken, orally, according to a dosage that facilitates compliance. It is in fact advantageously configured to be taken in a quantity equal to two tablets per day. According to a particularly advantageous method of administration, the two tablets a day must be taken at the same time.

Preliminary tests demonstrate the beneficial effects of the dietary supplement according to the invention.

In particular, an "in vitro" comparative test was conducted to demonstrate the anti-inflammatory potential of the claimed supplement, with a specific effect on the main mediators involved in the painful manifestation of the pathology in question.

The test was conducted in the following way.

Fibroblast cell lines were treated with an inflammatory stimulus, for example the lipopolysaccharide LPS (one of the components of the outer layer of the cell wall of Gram-negative bacteria) or zymosan (commercial preparation of yeast cell wall nanoproteins) the claimed supplement was applied to said culture. In detail, the equivalent of a daily dose, i.e. one or two tablets per day, of a powder obtained from ten of the claimed tablets was applied to the culture, said powder thus containing overall:
- 3000 mg of Palmitoylethanolamide;
- 1500 mg freeze-dried Noni juice powder;
- 1500 mg of Boswellia resinous extract 65% boswellic acids ;
- 1250 mg of REPHYLL ^{®} beta-caryophyllene ;
- 1000 mg of MYRLIQ ^{®};
- 500 mg of Griffonia extract with 25% 5-hydroxy-tryptophan;
- 500 mg of feverfew dry extract 0.5% Parthenolides;
- 2350 mg of microcrystalline cellulose;
- 200 mg of silicon dioxide; and
- 200 mg of Mg salts of fatty acids.

Administering a portion of a powder obtained from several tablets, rather than directly administering the powder of one or two tablets, has the aim of minimizing the variation, compared to the nominal values, in the actual quantity of the active ingredients in the tablets caused by tolerances of production.

In the culture medium thus obtained, the release of inflammatory cytokines was measured within 24 hours of treatment, in particular at least one of interleukin IL-1, interleukin IL-6 and tumor necrosis factor α (TNF-α).

As a comparative reference, a culture of fibroblast cell lines was used, to which the same inflammatory stimulus was applied, and was subsequently treated with a supplement known by the trade name of VULVOLEN^{®}. In particular, the culture was administered the equivalent of a daily dose, i.e. one or two tablets per day, of a powder obtained from 30 tablets of 1000 mg of VULVOLEN^{®}, where each tablet includes the following active ingredients:
- 200 mg of alpha lipoic acid;
- 50 mg of PEA;
- 5.5 mg Thiamine;
- 4.2 mg of Vitamin B6; and
- 25 mcg of Vitamin B12.

From the measurement of the inflammatory chitins of the VULVOLEN^{®} reference sample and the comparison with the inflammatory chitins measured in the culture treated with the claimed supplement, it emerged that in the culture treated with the claimed supplement the release of inflammatory chitins was lower than in the sample reference, thus confirming the inflammation-reducing effect of the supplement.

The testing methodology described above is based on the following considerations.

Observational studies have examined the expression of pro-inflammatory mediators in the vulvar vestibule and detected a higherTNF-α concentration in women with vulvodynia ^{[9]}.

Foster and Hasday found elevated tissue levels of IL-1β and TNF-α, observing that the concentration of these pro-inflammatory mediators was actually higher in the surrounding vulvar tissue than in the specific area of inflammation and confirming what emerged from clinical observations, according to which the area involved in the inflammatory process is much larger than the area in which the irritative state is observed ^{[10]}.

Some studies have highlighted polymorphisms in the genes that regulate the inflammatory response, indicating a possible genetic involvement. As with common inflammatory conditions, allele 2 of the IL-1β gene was found in significantly more women diagnosed with localized provoked vulvodynia (LPV) (40%) compared to controls (25%) ^{[11]}.

Fibroblast cell lines taken from patients affected by LPV revealed a complex immune mechanism underlying vulvar pain, highlighting an increase in the release of inflammatory cytokines following stimulation with yeast antigens in vitro (the trigger cited by over 70% of patients appears to be a series of recurrent yeast infections), as well as a correlation between the increased release of interleukin IL-6 and prostaglandin E2 (PGE2) and hypersensitivity to contact and pain ^{[12,13]}.

Overall, the scientific evidence therefore suggests that at the basis of chronic vulvar pain there may be alterations in the inflammatory responses of tissue fibroblasts, potentially triggered by an increase in the mast cell population .

For this reason, in order to demonstrate the effectiveness of the claimed supplement in the treatment of the disorder in question, the ability of the supplement to reduce the release of pro-inflammatory mediators associated with pain IL-6, IL-1β and TNF-α by fibroblasts, considering the scientific evidence that shows that in the pathology in question there is an increase in the release of the aforementioned molecules.

In addition to the in vitro comparative test, a clinical test was performed on 17 subjects, resulting in, with a power of 80%, a probability of type I alpha error equal to 0.05 and with an effect size (d) equal to 0.827, that the minimum numerousness was equal to 15 subjects.

The subjects were selected according to the following inclusion criteria:
- female sex;
- age between 18 and 70;
- general state of good health;
- diagnosis of vulvodynia; and
- subjective evaluation of dyspaurenia greater than or equal to 2 on the VAS scale.

The following were excluded:
- subjects who are pregnant, breastfeeding or planning to become pregnant before the end of the study;
- subjects with other concomitant pathologies such as cancer, fibromyalgia, chronic intestinal inflammatory diseases, autoimmune diseases;
- subjects with proven sensitivity to one of the components;
- subjects who require the prescription of a new therapy compared to the one already underway at the time of enrollment;
- subjects who have any other condition that, in the opinion of the investigator, could compromise the safety or availability of the subject or adherence to the study protocol or could interfere with the interpretation of the results and would therefore make the subject inappropriate for the study inclusion in monitoring; and
- subjects who are not self-sufficient in the application of the medical device.

The clinical study lasted 18 weeks starting from a first visit in which the following occurred: baseline visit, enrollment, collection of self-assessments expressed via VAS (Visual Analogue Scale), completion of the SF-36 questionnaire (quality assessment questionnaire of life) for quality of life, and assignment of treatment with the claimed supplement.

Subsequently, 60 days after the start of treatment with the claimed supplement, the self-assessments expressed via VAS scale were collected, the SF-36 questionnaire for quality of life was completed and any adverse events were recorded.

At the end of 18 weeks from the start of treatment with the claimed supplement, the self-assessments expressed via VAS scale were collected, the SF-36 questionnaire for quality of life was completed and any adverse events were recorded.

The self-assessments include: self-assessment of dyspaurenia on a VAS scale from 0 to 10, self-assessment of spontaneous vestibular pain on a VAS scale from 0 to 10, self-assessment of provoked vestibular pain on a VAS scale from 0 to 10, self-assessment of symptoms from vulvo -vaginal infection in VAS scale from 0 to 10.

The clinical tests showed for the sample subjected to treatment with the claimed supplement a reduction in the value on the VAS scale in the self-assessment of dyspaurenia, a reduction in the value on the VAS scale in the self-assessment of spontaneous vestibular pain, a reduction in the value on the VAS in the self-assessment of provoked vestibular pain, a reduction in the VAS scale value in the self-assessment of symptoms from vulvo-vaginal infection, and an improvement in the quality of life based on the completion of the SF-36 questionnaire.

In the foregoing, the preferred embodiments have been described and variations of the present invention have been suggested, but it is to be understood that those skilled in the art will be able to make modifications and changes without thereby departing from the relevant scope of protection, as defined by the claims. attached.

### BIBLIOGRAPHY

[1]. Rosen NO, Dawson SJ, Brooks M., Kellogg-Spadt S.; Treatment of Vulvodynia: Pharmacological and Non-pharmacological Approaches, Springer Nature Switzerland, 2019, 79:483-493.
[2]. Vasileva P., Strashilov SA, Yordanov AD; Aetiology, diagnosis, and clinical management of vulvodynia, Menopause, Rev 2020; 19(1): 44-48.
[3] Arnold, L.D.; Bachmann, G.A.; Rosen, R.; Kelly, S.; Rhoads, GG Vulvodynia: Characteristics and associations with comorbidities and quality of life. Obstet. Gynecol. 2006, 107, 617-624.
[4] Latthe , P.; Mignini, L.; Gray, R.; Hills, R.; Khan, K. Factors predisposing women to chronic pelvic pain: Systematic review. BMJ 2006, 332, 749-755.
[5] Dussossoy , E.; Brat, P.; Bony, E.; Boudard , F.; Poucheret , P.; Mertz, C.; Giaimis , J.; Michel, A. Characterization, Anti-Oxidative and Anti-Inflammatory Effects of Costa Rican Noni Juice ( Morinda citrifolia L.). J. Ethnopharmacol . 2011, 133, 108-115.
[6] MZ SIDDIQUI*, Boswellia Serrata, A Potential Antiinflammatory Agent: An Overview, May-June 2011, Indian Journal of Pharmaceutical Sciences ,255-261.
[7] P. Dolara , C. Luceri , C. Ghelardini et al., "Analgesic effects of myrrh [5]," Nature, vol. 379, no. 6560, p. 29, 1996.
[8] Germano A., Occhipinti A., Barbero F., Maffei ME A Pilot Study on Bioactive Constituents and Analgesic Effects of MyrLiq, a Commiphora myrrha Extract with a High Furanodiene Content, Clinical Study, BioMed Research International, Volume 2017, Article ID 3804356, 11 pages, https://doi.org/10.1155/2017/3804356
[9] Seckin -Alac E, Akhant SE, Bastu E, Tuzlalik S, Yavuz E. Elevated tissue levels of tumor necrosis factor-alpha in vulvar vestibulitis syndrome. Clinical and experimental obstetrics & gynecology . 2014; 41(6):691-3.
[10] Foster DC, Hasday JD. Elevated tissue levels of interleukin-1-beta and tumor necrosis factor alpha in vestibulitis vulvar.Obstet Gynecol . 1997; 89:291-6.
[11] Gerber S , Bongiovanni AM, Ledger WJ, Witkin SS. Interleukin-1beta gene polymorphism in women with vulvarvestibulitis syndrome. Eur J Obstet Gynecol Reprod Biol. 2003;107:74-7.
[12] Foster, D.C., Falsetta, M.L., Woeller, C.F., Pollock, S.J., Song, K., Bonham, A., et al. (2015). Site-specific mesenchymal control of inflammatory pain to yeast challenge in vulvodynia-afflicted and pain-free women. Pain 156, 386-396. doi :10.1097/01.j.pain.0000460320.95267.5d;
[13] Foster, D.C., Piekarz, K.H., Murant, T.I., LaPoint, R., Haidaris , C.G., Phipps, R.P. (2007). Enhanced synthesis of proinflammatory cytokines by vulvar vestibular fibroblasts: implications for vulvar vestibulitis. Am . J. Obstet. Gynecol . 196, 346.e1-346.e8.

## Claims

1. Dietary supplement comprising or consisting of:
- Palmitoylethanolamide, in percentual weight comprised between 20% and 30%;
- Noni juice freeze-dried powder, in percentual weight comprised between 10% and 15%;
- Boswellia resinous extract comprising about 65% of boswellic acids, in percentual weight comprised between 10% and 15%;
- β- caryophyllene, in percentual weight comprised between 8.32% and 12.5%;
- Myrrh extract, in percentual weight comprised between 6.64% and 9.96%;
- Griffonia seed dry extract comprising about 25% of 5-hydroxy-tryptophan, in percentage weight between 3.36% and 5.04%;
- parthenium dry extract comprising about 0.5% of Parthenolides, in percentual weight comprised between 3.36% and 5.04%;
- microcrystalline cellulose, in percentual weight comprised between 15.7% and 23.16%;
- silicon dioxide, in percentual weight comprised between 1.66% and 1.99%; And
- Mg salts of fatty acids, in percentual weight comprised between 1.66% and 1.99%,
in which the total percentage sum of all the aforementioned components is equal to 100%.

2. Dietary supplement according to claim 1, wherein the percent weight concentration of Palmitoylethanolamide is equal to 25%.

3. Dietary supplement according to claim 1 or 2, wherein the percent weight concentration of the freeze-dried Noni juice powder is equal to 12.5%.

4. Dietary supplement according to any of the previous claims, in which the percentage weight concentration of the Boswellia resinous extract comprising about 65% of boswellic acids is equal to 12.5%.

5. Dietary supplement according to any of the previous claims, in which the percentage weight concentration of β- caryophyllene is equal to 10.4%.

6. Dietary supplement according to any of the previous claims, in which the percentage weight concentration of Myrrh extract, optionally in the form of oleoresin, is equal to 8.3%.

7. Dietary supplement according to any of the previous claims, wherein the percentage weight concentration of Griffonia seed dry extract comprising about 25% of 5-hydroxy-tryptophan is equal to 4.2%.

8. Dietary supplement according to any of the previous claims, in which the percentage weight concentration of Parthenium dry extract comprising about 0.5% of Parthenolide is equal to 4.2%.

9. Dietary supplement according to any of the previous claims, having the form of a tablet weighing 1200 mg, said tablet consisting of:
- 300 mg Palmitoylethanolamide;
- 150 mg freeze-dried Noni juice powder;
- 150 mg of Boswellia resinous extract comprising about 65% boswellic acids ;
- 125 mg of β-caryophyllene , in particular REPHYLL ^{®} beta-caryophyllene ;
- 100 mg of Myrrh extract, in particular MYRLIQ ^{®} myrrh gum;
- 50 mg of Griffonia seed dry extract comprising about 25% 5-hydroxy-tryptophan ;
- 50 mg of parthenium dry extract including about 0.5% parthenolides ;
- 235 mg microcrystalline cellulose;
- 20 mg of silicon dioxide; And
- 20 mg of Mg salts of fatty acids.

10. Dietary supplement according to any of the previous claims, for use in the prevention and/or treatment of vulvodynia.

11. Dietary supplement according to claims 9 and 10, configured to be administered orally in the form of two tablets per day.

12. Dietary supplement according to claim 11, wherein the two tablets are taken at the same time.
